# EUROPÄISCHE PATENTSCHRIFT

(11) **EP 0 323 573 B1**
(45) Veröffentlichungstag und Bekanntmachung des Hinweises auf die Patenterteilung: **03.02.1993**
(21) Anmeldenummer: 88119951.7
(22) Anmeldetag: 30.11.1988
(51) Int. Cl.: C07C 209/26, C07C 211/03

(54) **Verfahren zur Herstellung von tertiären N,N-Dimethylaminen**
Process for the preparation of tertiary N,N-dimethyl amines
Procédé pour la préparation de N,N-diméthylamines tertiaires

(30) Priorität: 09.12.1987 DE 3741726
(43) Veröffentlichungstag der Anmeldung: 12.07.1989
(73) Patentinhaber: HOECHST AKTIENGESELLSCHAFT, 65926 Frankfurt am Main (DE)
(72) Erfinder: Weber, Jürgen, Dr. Dipl.-Chem., D-4200 Oberhausen 11 (DE); Kampmann, Detlef, Dr. Dipl.-Chem., D-4630 Bochum 6 (DE); Kniep, Claus, D-4200 Oberhausen 11 (DE)

(56) Entgegenhaltungen:
- EP-A- 0 142 868
- EP-A- 0 297 295
- FR-A- 2 349 565
- GB-A- 436 414

## Beschreibung

Die vorliegende Erfindung betrifft ein Verfahren zur Herstellung von tertiären N,N-Dimethylaminen durch Umsetzung primärer Amine mit Formaldehyd und Wasserstoff an einem Hydrierkatalysator in flüssiger Phase. Als Amine können beliebige einwertige oder mehrwertige primäre Amine sowie Mischungen derselben eingesetzt werden. Die Umsetzung läuft bei erhöhter Temperatur ab und erfordert in der Regel einen erhöhten Druck. Sie führt zu einem vollständigen Ersatz aller am Aminstickstoffatom befindlichen Wasserstoffatome, die unter Bildung von Wasser gegen jeweils eine Methylgruppe ausgetauscht werden. Man bezeichnet diese Reaktion auch als hydrierende N-Methylierung von Aminen.

Tertiäre Amine stellen technisch bedeutsame Verbindungen dar. Sie können als Polymerisations- und Härtungskatalysatoren für die Herstellung von Kunststoffen auf Epoxid- und Urethanbasis dienen. Ferner sind sie als Korrosionsinhibitoren und Adsorptionsmittel für die Synthesegaswäsche geeignet. Das trifft besonders auf die leicht herstellbaren Dimethylderivate zu.

Die hydrierende Methylierung von primären Aminen mit Formaldehyd und Wasserstoff ist ein wichtiger Syntheseweg zur Herstellung methylierter Amine. Eine Zusammenfassung dieses Verfahrens findet sich in Houben-Weyl, Methoden der organischen Chemie; Band XI/1, Seiten 641 bis 643, 4. Auflage (1957).

Gemäß DE-OS 19 32 422 kann man die Umsetzung unter Verwendung üblicher, festangeordneter Katalysatoren in flüssiger Phase durchführen.

Die ältere Anmeldung DE 37 21 539 beschreibt ein modifiziertes Verfahren. Hierin werden die Ausgangsstoffe Amin, Formaldehyd und Wasserstoff voneinander getrennt auf eine vorgegebene Temperatur erhitzt und anschließend in Gegenwart eines festangeordneten Katalysators zusammengeführt. Die Ausgangsstoffe, insbesondere der Formaldehyd sollen einen verminderten Wasseranteil enthalten. Die festangeordneten Katalysatoren sind in Rohrreaktoren, die den erforderlichen hohen Drücken standhalten, untergebracht.

Es ist jedoch auch möglich auf den Gebrauch aufwendig konstruierter Hochdruckrohrreaktoren zu verzichten und die Umsetzung beispielsweise in Rührbehältern oder Schlaufenreaktoren durchzuführen. In diesem Falle wird die hydrierende N-Methylierung mit Hilfe eines aufgeschlämmten Hydrierkatalysators durchgeführt. Dieses Verfahren nennt man auch Suspensionsfahrweise. Die vorliegende Erfindung bezieht sich auf diese Verfahrensvariante.

Bei längerem Gebrauch zerfallen die Hydrierkatalysatoren in zunehmendem Maße. Die resultierenden, feinteiligen Partikel sind nicht erwünscht, da sie die Abtrennung des suspendierten Katalysators nach der Umsetzung erschweren. Üblicherweise entfernt man den aufgeschlämmten Katalysator durch Sedimentation und/oder Zentrifugieren und/oder Filtration. Je feiner die Katalysatorpartikel sind, desto schwerer lassen sie sich aus dem Reaktionsgemisch entfernen. Eine geringere Teilengröße bedeutet eine verminderte Sedimentationsgeschwindigkeit, was sich auch auf das Zentrifugieren nachteilig auswirkt. Verwendet man die Filtration zur Katalysatorabtrennung, so führen besonders kleine Teilchen infolge Verlegung der Filterporen rasch zu einer Blockierung der Filtrationseinheit. Dadurch kommt es zu einem Druckanstieg in der Apparatur, mit der Folge, daß die Filtration unterbrochen werden muß, um die Verstopfungen zu beseitigen.

Unerwünscht ist auch die Entstehung von Ameisensäure, die sich vermutlich durch Cannizzaro-Reaktion aus Formaldehyd bildet. Sie entzieht der Reaktion eine entsprechende Menge Amin als Salz. Die freie Säure wie auch das Aminsalz begünstigen Korrosionen im Reaktorsystem.

Weitere, störende Nebenreaktionen beruhen auf der Polymerisation von Formaldehyd mit sich selbst und der Polykondensation zwischen Amin und Formaldehyd zu Hexahydrotriazinen oder im Falle mehrwertiger Amine zu höhermolekularen Verbindungen. Die Bildung polymerer Stoffe zieht infolge von Verklebungen des suspendierten Kontaktes eine Verminderung der Katalysatoraktivität nach sich und kann bis zu einer Blockierung des Reaktors führen. Ein Kontaktwechsel ist die unvermeidliche Folge.

Die EP-A 0 142 868 empfiehlt für die N-Alkylierung von Aminen die Verwendung spezieller Hydrierkatalysatoren, die wenigstens eines der Elemente Co, Ni, Ru, Rh, Pd und Pt auf Kohle als Träger enthalten. Der Katalysator wird in dem Amin suspendiert und die Carbonylverbindung kontinuierlich zugesetzt. Aluminiumoxid, Siliciumdioxid und Kieselerde sind als Träger für die Hydrierkatalysatoren unerwünscht, da sie eine gute Verteilung des Katalysators im Reaktionssystem nicht sicherstellen. Metallkatalysatoren ohne Träger wie Raney-Nickel, Raney-Kobalt, Palladium- und Platinschwarz sind zwar sehr aktiv, liefern aber keine guten Ergebnisse, wie Vergleichsversuche belegen.

Zu einem vergleichbaren Befund führt die ES 538 216. Während Pd- und Pt-haltige Katalysatoren auf Aktivkohle gute Ergebnisse liefern, führen - bestätigt durch Vergleichsversuche - Raney-Nickel und ein üblicher Nickel-Trägerkatalysator bei der N-Methylierung primärer Amine zu recht ungünstigen Resultaten.

Wie aus den vorangegangenen Ausführungen hervorgeht, ist ein erfolgreicher Einsatz von Nickel enthaltenden Katalysatoren für die Umsetzung von primären Aminen mit Formaldehyd und Wasserstoff lediglich auf einen speziellen Nickel-Aktivkohle-Trägerkatalysator beschränkt.

Überraschenderweise wurde gefunden, daß die N-Methylierung primärer Amine mittels beliebiger Nickel enthaltender Katalysatoren ohne nennenswerte Minderung an Umsatz und Selektivität durchgeführt werden kann. Die Reaktion liefert bei nahezu quantitativem Umsatz destillative Ausbeuten von 90 % und mehr bezogen auf eingesetztes Amin.

Gelöst werden diese Aufgaben durch ein Verfahren zur Herstellung von tertiären N,N-Dimethylaminen durch Umsetzung von primären Aminen mit Formaldehyd und Wasserstoff als Ausgangsstoffe unter Druck und erhöhter Temperatur an einem Nickel enthaltenen Hydrierkatalysator in flüssiger Phase, dadurch gekennzeichnet, daß der Hydrierkatalysator in einem Lösungsmittel suspendiert wird, die Nickel-Konzentration 0,1 bis 10 Gew.-%, bezogen auf einzusetzendes primäres Amin, beträgt, die Ausgangsstoffe bei 80 bis 150 °C und 1 bis 15 MPa voneinander getrennt, aber gleichzeitig unter Vermischung in die Katalysatorsuspension eingeleitet und in einem Reaktionsschritt zu tertiären N,N-Dimethylaminen umgesetzt werden.

Die Ausgangsstoffe werden entsprechend einer bewährten Arbeitsweise bei 90 bis 130, insbesondere bei 95 bis 125, bevorzugt bei 100 bis 120 °C umgesetzt. Der Druck beträgt 1,5 bis 12 MPa, insbesondere 3 bis 10 MPa, bevorzugt 5 bis 8 MPa.

Der Hydrierkatalysator soll nicht weniger als 10 Gew.-% Ni bezogen auf gesamten Katalysator aufweisen. Hydrierkatalysatoren mit mindestens 20 Gew.-% Ni bezogen auf gesamten Katalysator sind gut geeignet. Hierunter fallen eine Reihe handelsüblicher Trägerkatalysatoren mit 20 bis 80, insbesondere 40 bis 70, bevorzugt 50 bis 65 Gew.-% Ni bezogen auf gesamten Katalysator. In vielen Fällen wird man Katalysatoren mit mindestens 40 Gew.-% Ni bezogen auf gesamten Katalysator benutzen.

Die Nickel enthaltenden Katalysatoren können Trägerstoffe besitzen, aber auch frei von Trägern sein. Sie enthalten neben Ni gegebenenfalls gebräuchliche Zusatzstoffe und/oder Promotoren, beispielsweise Erdalkalioxide, SiO₂, Al₂O₃, MnO₂ und/oder Cr₂O₃.

Vorteilhaft ist der Gebrauch von Trägerkatalysatoren. Als Träger dienen Al₂O₃, SiO₂, Kieselerde, Kieselgel, Aktivkohle und/oder Bimsstein, insbesondere Al₂O₃, SiO₂, Kieselerde und/oder Kieselgel bevorzugt SiO₂, Kieselerde und/oder Kieselgel.

Das erfindungsgemäße Verfahren ist unabhängig von der Art der verwendeten primären Amine. Es läßt sich allgemein auf sämtliche organische Verbindungen mit einer oder mehreren primären Amingruppen anwenden. Als Amine lassen sich einwertige und/oder mehrwertige primäre Amine verarbeiten. Aliphatische oder cycloaliphatische Amine können ebenso in die erfindungsgemäße N-Methylierung eingesetzt werden wie araliphatische, aromatische oder heterocyclische Amine. Auch Mischungen derselben lassen sich verarbeiten.

Die primären Amine besitzen insgesamt 1 bis 40 Kohlenstoffatome. Die vorhandenen Substituenten können geradkettige und/oder verzweigte Alkylreste mit 1 bis 24 Kohlenstoffatomen, unsubstituierte und/oder substituierte Cycloalkylreste mit 5 bis 20 Kohlenstoffatomen, unsubstituierte und/oder substituierte aromatische Gruppen mit 6 bis 20 Kohlenstoffatomen oder heterocyclische Reste mit 4 bis 20 Kohlenstoffatomen, die als Heteroatom Sauerstoff, Schwefel und/oder Stickstoff enthalten können, sein.

Beispiele für primäre aliphatische Amine sind: Methylamin, Ethylamin, Propylamin, n- und i-Butylamin, 3-Methylbutylamin, n-Pentylamin, 2-Methylpentylamin, n-Hexylamin, n-und i-Heptylamin, n- und i-Octylamin, n- und i-Nonylamin, n- und i-Decylamin, n- und i-Undecylamin, 2-Methylundecylamin, n-Dodecylamin, n- und i-Tridecylamin, n- und i-Hexadecylamin, Stearylamin, Cerylamin, Ethylendiamin, 1,3-Diaminopropan, 1,4-Diaminobutan, 1,6-Diaminohexan (Hexamethylendiamin-(1,6)), ω ,ω′-Polyalkylendiamine, Aminoalkohole wie Ethanolamin, Propanolamin, Diglykolamin.

Als Beispiele für cycloaliphatische Amine seien genannt: Cyclopentylamin, Cyclohexylamin und Tricyclodecanamine. Die Ringsysteme können Substituenten, insbesondere Alkylreste, aufweisen.

Beispiele für araliphatische Amine sind: Benzylamin, alpha-und beta-Phenylethylamin und Phenylpropylamin.

Beispiele für aromatische Amine sind: Anilin, Toluidin, Benzidin (4.4′-Diaminodiphenyl), Phenylendiamine, substituierte und unsubstituierte Naphthylamine.

Das erfindungsgemäße Verfahren hat sich besonders bei der N-Methylierung, geradkettiger und/oder verzweigter primärer aliphatischer Amine bewährt.

Primäre aliphatische Amine neigen mit steigender Zahl der C-Atome dazu, in Gegenwart Nickel enthaltender Hydrierkatalysatoren unter Bildung entsprechend substituierter Di- und Trialkylamine umzualkylieren.

Bei Anwendung des erfindungsgemäßen Verfahrens treten diese unerwünschten Übertragungen eines Alkylrestes von einem Aminstickstoffatom auf ein anderes nicht auf. Daher eignet sich diese Arbeitsweise vorzüglich zur Umsetzung primärer, aliphatischer Amine mit 4 bis 24, insbesondere 6 bis 20, bevorzugt 8 bis 16 Kohlenstoffatomen.

Der Nickel enthaltende Katalysator kann in stückiger Form beispielsweise als Fadenkorn, Tabletten, Pellets oder Granulat, aber auch in zerkleinertem Zustand oder als Pulver eingesetzt werden. Der Körnungsgrad soll nicht zu grob aber auch nicht zu fein sein. Zu große Katalysatorpartikel wirken sich infolge einer zu geringen Oberfläche, an der die Reaktion abläuft, ungünstig aus. Hingegen erweisen sich feinteilige Katalysatoren aufgrund der großen Oberfläche als reaktiv, lassen sich aber wegen der geringen Teilchengröße schlecht durch Sedimentation, Zentrifugieren oder Filtration abtrennen. Die Entfernung des Nickel enthaltenden Katalysators nach Abschluß der Umsetzung ist notwendig, um unerwünschte Nebenreaktionen in der Reindarstellung zu vermeiden. Während der Destillation führen bereits geringe Nickelanteile zu Umlagerungen und Umalkylierungen. Die dabei gebildeten Nebenprodukte verunreinigen das gewünschte tertiäre N,N-Dimethylamin.

Vor Beginn der Reaktion wird der Nickel enthaltende Hydrierkatalysator in einem Lösungsmittel suspendiert. Dieses Solvens soll gegenüber der ablaufenden Umsetzung inert sein, d. h. es darf nicht in die N-Methylierung als Reaktionspartner eingreifen.

Als Lösungsmittel sind das Reaktionsprodukt, aliphatische, cycloaliphatische, aromatische Kohlenwasserstoffe, Ether und/oder Alkohole geeignet.

In vielen Fällen haben sich cyclische Ether und/oder aliphatische Alkohole bewährt.

Besonders brauchbar sind Tetrahydrofuran, Dioxan und/oder aliphatische Alkohole mit 1 bis 6 Kohlenstoffatomen, insbesondere Methanol, Ethanol, Propanol, i-Propanol, n-Butanol und/oder i-Butanol.

Wegen guter Wasserlöslichkeit sind Methanol, Ethanol und/oder Propanole, insbesondere Methanol und/oder Ethanol bevorzugt Methanol als Lösungsmittel zu empfehlen.

Das Lösungsmittel ist in Abhängigkeit von dem jeweils umzusetzenden primären Amin auszuwählen. Amine mit relativ hohem Molekulargewicht erfordern mitunter Lösungsmittel geringer Polarität, z. B. Benzol, Toluol und/oder Xylol, aber auch Gemische verschiedener Lösungsmittel können verwendet werden. Um die erforderliche Polarität einzustellen, kann es notwendig sein, unpolare Lösungsmittel mit polaren Solventien zu versetzen.

Bei der Umsetzung primärer Amine mit Formaldehyd und Wasserstoff wird Wasser gebildet. Neben diesem Reaktionswasser gelangt zusätzlich Wasser mit den Einsatzstoffen, insbesondere mit dem Formaldehyd in die Umsetzung.

Es empfiehlt sich den Wassergehalt zu begrenzen. Er sollte am Ende der Reaktion höchstens 35, insbesondere 25 bevorzugt 15 Gew.-% bezogen auf gesamtes Reaktionsgemisch betragen. Zu hohe Wasseranteile können einerseits zur Ausbildung einer heterogenen Wasserphase führen, andererseits kann der Nickel enthaltende Katalysator geschädigt werden.

Eine heterogen anfallende Wasserphase lagert sich unten im Reaktor ab und verhindert die Umsetzung zumindest in dem Bereich, wo sie den Katalysator einschließt. Dieser vom Wasser umhüllte Katalysatoranteil steht für die Reaktion nicht mehr zur Verfügung.

Ein zu hoher Wassergehalt führt insbesondere über längere Zeit zu einer Beeinträchtigung der Katalysatoraktivität und verringert sowohl Umsatz als auch Selektivität der Reaktion. Außerdem wird ein rascher Zerfall des Katalysators begünstigt. Dies trifft in großem Maße auf eine Reihe von Trägerkatalysatoren zu. Offenbar greift das Wasser das Trägermaterial an und setzt seine Haltbarkeit schnell herab.

Ein rascher Zerfall des suspendierten Kontaktes ist wegen der schlechten Abtrennbarkeit feinteiliger Katalysatorpartikel nachteilig.

Die Begrenzung des Wasseranteils in der Reaktionsmischung kann einerseits durch Art und Menge des Lösungsmittels und andererseits durch den einzusetzenden Formaldehyd, der etwa 60 Gew.-% und mehr Wasser bezogen auf Formaldehydlösung enthalten kann, erreicht werden.

Lösungsmittel, die Wasser in begrenztem oder hohem Umfange zu lösen vermögen, verhindern die Ausbildung einer heterogenen Wasserphase. Ihr Anteil sollte mindestens 5 Vol.-% bezogen auf das Volumen aller flüssigen Ausgangsstoffe betragen, aus Gründen der Wirtschaftlichkeit jedoch nicht über 50 Vol.-% liegen.

In vielen Fällen wird ein Lösungsmittelzusatz von 5 bis 40, insbesondere 10 bis 30, bevorzugt 15 bis 25 Vol.-% bezogen auf das Volumen aller flüssigen Ausgangsstoffe genügen. Fällt je Volumteil Amin wenig Reaktionswasser an, so reicht ein Zusatz von 5 bis 20 Vol.-%, wird mehr Reaktionswasser je Volumteil freigesetzt, empfiehlt sich eine Zugabe von 10 bis 30 oder 15 bis 35 Vol.-% bezogen auf das Volumen aller flüssigen Ausgangsstoffe.

Das Lösungsmittel wird zumindest teilweise mit dem Nickel enthaltenden Hydrierkatalysator in dem Reaktor vorgelegt. Weitere Anteile können mit einem der Ausgangsstoffe vorzugsweise mit Formaldehyd, der Reaktion zugeführt werden.

Besonders bewährt hat sich die Verwendung eines wasserarmen, lösungsmittelhaltigen Formaldehyds. Derartige Lösungen bestehen aus Formaldehyd, 5 bis 15, insbesondere 7 bis 12 Gew.-% Wasser und 25 bis 55, insbesondere 30 bis 40 Gew.-% eines aliphatischen Alkohols, insbesondere Methanol.

Gut geeignet ist eine Lösung, die etwa 55 Gew.-% Formaldehyd, 10 Gew.-% Wasser und 35 Gew.-% Methanol aufweist. Ein Produkt dieser Zusammensetzung befindet sich im Handel.

Man kann das Lösungsmittel auch mit dem Amin der Reaktion zuleiten. Dies empfiehlt sich besonders bei pastösen oder festen, primären Aminen höheren Molekulargewichts.

Üblicherweise führt man die Reaktion in einem druckfesten Behälter durch. Die notwendige Durchmischung kann durch Kreislaufführung des suspendierten Hydrierkatalysators und/oder durch Rühren erfolgen. In vielen Fällen kann ein üblicher Rührwerkbehälter verwendet werden. Die Reaktion kann kontinuierlich oder diskontinuierlich durchgeführt werden, sie eignet sich insbesondere für eine diskontinuierliche Arbeitsweise.

Der gegebenenfalls zerkleinerte, Nickel enthaltende Hydrierkatalysator wird zusammen mit Lösungsmittel in den Druckbehälter gegeben und durch Mischen suspendiert. Die Suspension wird auf 80 bis 150 °C erwärmt. Durch Zugabe von Wasserstoff wird ein Druck von 1 bis 15 MPa eingestellt.

Ein wesentliches Merkmal der Erfindung ist es, daß die Ausgangsstoffe voneinander getrennt, aber gleichzeitig unter Vermischung in die Katalysatorsuspension eingeleitet werden.

Das bedeutet, daß man Amin, Formaldehyd und Wasserstoff über eigene Leitungen der Reaktionszone, in der sich der suspendierte Katalysator befindet, zusetzt.

Man kann auch den Wasserstoff im Gemisch mit Amin oder im Gemisch mit Formaldehyd der Reaktion zuleiten. Es ist ferner möglich, den Wasserstoff auf Amin und Formaldehyd zu verteilen.

Alle drei Ausgangsstoffe Amin, Formaldehyd und Wasserstoff dürfen erst in Gegenwart des suspendierten Katalysators aufeinandertreffen.

Wird der Wasserstoff zusammen mit einem der übrigen Ausgangsstoffe der Reaktion zugeleitet, so dürfen das Amin und der Formaldehyd erst in Anwesenheit des suspendierten Nikkel enthaltenden Katalysators zusammengeführt werden. Auch in diesem Falle geraten alle drei Ausgangsstoffe nur im Beisein des Kontaktes miteinander in Berührung.

Es ist möglich, die Leitungen zweier oder auch aller drei Ausgangsstoffe in der Zone des suspendierten Katalysators münden zu lassen. Welche Anordnung der Leitungen zu wählen ist, wird zum einen von den Stoffmengen und zum anderen von der Geometrie der Reaktionszone und den erforderlichen Strömungsverhaltnissen abhängen. Bei Durchsatz größerer Stoffmengen je Zeiteinheit empfiehlt es sich, je Ausgangsstoff nicht nur eine, sondern mehrere Leitungen zu gebrauchen. Sollten die Strömungsverhältnisse eine ausreichende Durchmischung der Reaktanten in der Katalysatorzone nicht sicherstellen, ist die Verwendung zusätzlicher Verteilervorrichtungen nützlich. Diese Verteiler können beispielsweise als Ringbrause oder Duschkopf am Ende einer Leitung angebracht werden. Aber auch andere Verteilersysteme wie Düsen, Fritten oder Röhrenbündel können Verwendung finden.

Gemäß einer besonderen Ausführungsform der Erfindung führt man primäres Amin und Formaldehyd über zwei separate Tauchrohre in die Katalysatorsuspension ein. Der Wasserstoff kann entweder über ein zusätzliches Tauchrohr in die Reaktionszone oder über einen Rohrstutzen in den über der Suspension befindlichen Gasraum eingeleitet werden.

Die Reaktionsbedingungen, insbesondere der Druck, die Temperatur und die Reaktionsdauer, hängen in gewissem Umfange auch von der Art des primären Amins und dem suspendierten Hydrierkatalysator ab.

Amine geringer thermischer Stabilität setzt man bei 80 bis 100 °C, Amine mit mittlerer Reaktivität bei 100 bis 120 °C und solche mit geringem Reaktionsvermögen bei 120 bis 150 °C um.

Nickel enthaltende Katalysatoren mit hoher Aktivität lassen eine Reaktion bei relativ niedrigen Temperaturen zu, während Kontakte mit mittlerer Aktivität höhere Temperaturen erfordern.

Die Aktivität eines Katalysators vermindert sich in Abhängigkeit von der Betriebsdauer. Katalysatoren, die vielfach in die hydrierende N-Methylierung wieder eingesetzt wurden, benötigen im Laufe der Zeit immer höhere Reaktionstemperaturen, um einen möglichst optimalen Ablauf der Synthese zu gewährleisten. Sogar ursprünglich sehr aktive Katalysatoren können Reaktionstemperaturen von 140 °C und darüber erfordern.

Der Formaldehydbedarf richtet sich nach der Anzahl der am primären Aminstickstoff befindlichen Wasserstoffatome. Je Grammatom zu substituierenden Wasserstoff werden 1 bis 2 insbesondere 1 bis 1,5, bevorzugt 1,1 bis 1,25 Mol Formaldehyd eingesetzt. Im Regelfall wird ein stöchiometrischer Überschuß von 5 bis 30 Mol.-% Formaldehyd die Reaktion günstig beeinflussen.

Die benötigte Menge Wasserstoff wird durch die Stöchiometrie der Umsetzung vorgegeben. Üblicherweise setzt man den Wasserstoff in dem Maße zu, daß ein vorgegebener Druck eingehalten wird. Überschüssig eingesetzter Wasserstoff kann aus der Reaktion abgetrennt und der Umsetzung wieder zugeführt werden.

Die Zugabe von primärem Amin und Formaldehyd erfolgt voneinander getrennt, aber gleichzeitig. Wegen der Gefahr, daß unerwünschte Nebenprodukte gebildet werden, darf sie nicht zu schnell erfolgen. Eine etwas zu rasche Eindosierung von Amin und Formaldehyd kann durch eine Nachreaktion korrigiert werden, ohne daß nennenswerte Mengen an Nebenprodukten gebildet werden. In den meisten Fällen reicht hierfür eine Zeitspanne von 0,5 bis 2 Stunden aus. Eine zu langsame Zugabe kann allerdings die Bildung von Höhersiedern begünstigen.

Die Zugabegeschwindigkeit sollte für alle Einsatzstoffe, insbesondere für das primäre Amin und für Formaldehyd gleich sein. Sie hängt von verschiedenen Faktoren wie Größe des Ansatzes, Reaktivität des Amins, Art des Amins, Reaktorgeometrie, Katalysatorart, Nickelkonzentration, Intensität der Durchmischung, Druck und Temperatur ab. Sie ist den jeweiligen Bedingungen anzupassen und gegebenenfalls experimentell zu ermitteln. Hierbei ist der Verbrauch an Wasserstoff pro Zeiteinheit ein brauchbares Hilfsmittel, das Fortschreiten der Reaktion zu überprüfen. Erfolgt keine Wasserstoffaufnahme mehr, ist die Umsetzung abgeschlossen.

Die folgenden Beispiele belegen die Erfindung, ohne sie zu begrenzen.

### Beispiel 1:

Der Reaktorbehälter besteht aus einem 2 l Rührautoklaven, der neben einem Einlaß für die Wasserstoffzufuhr 2 Tauchstutzen besitzt.

In diesen Behälter werden 5 g eines pulverisierten Nickel-Katalysators (∼50 bis ∼53 Gew.-% Ni und etwa 25 bis 30 Gew.-% Kieselgur als Träger; Handelsprodukt der Hoechst AG: RCH Ni 52/35) und 100 g Methanol eingefüllt. Der pulverisierte Katalysator wird unter Rühren aufgeschlämmt und die Sollbedingungen (10 MPa Wasserstoffdruck; 120 °C) werden eingestellt.

Nach Erreichen der Sollbedingungen werden über die beiden Tauchstutzen gleichzeitig, aber getrennt 258 g (2 mol) n-Octylamin und 275 g einer Formaldehydlösung (∼55 Gew.-% Formaldehyd,∼ 10 Gew.-% Wasser und ∼35 Gew.-% Methanol) - entsprechend 5 mol Formaldehyd - über einen Zeitraum von 2 Stunden in die Katalysatorsuspension eingepumpt.

### Beispiel 2 bis 4

Es wird analog Beispiel 1 gearbeitet, jedoch die Einpumpzeit für die Einsatzstoffe n-Octylamin und Formaldehyd variiert. Sie beträgt 90 Minuten in Beispiel 2, 60 Minuten in Beispiel 3 und 30 Minuten in Beispiel 4.

### Beispiel 5:

Es wird analog Beispiel 1 gearbeitet, jedoch unter Verwendung von 5 g eines anderen pulverisierten Nickel-Katalysator (∼55 Gew.-% Nickel auf Kieselgur als Träger, Handelsprodukt der Hoechst AG: RCH Ni 55/5).

### Vergleichsversuch A

Unter Benutzung des in Beispiel 1 beschriebenen Reaktorbehälters, werden 258g (2 mol) n-Octylamin und 5 g des in Beispiel 1 verwendeten Nickelkatalysators vorgelegt. Auf die Zugabe von Methanol wird verzichtet.

Der pulverisierte Katalysator wird unter Rühren aufgeschlämmt und die Sollbedingungen (10 MPa Wasserstoffdruck, 120 °C) werden eingestellt.

Nach Erreichen der Sollbedingungen werden 275 g der in Beispiel 1 verwendeten Formaldehydlösung - entsprechend 5 mol Formaldehyd - über einen Zeitraum von 2 Stunden eingepumpt. Das Reaktionsprodukt besteht aus 2 Phasen, deren untere zu 98 Gew.-% aus Wasser und zu 2 Gew.-% aus N,N-Dimethyl-n-octylamin besteht. Die Zusammensetzung der organischen Phase ist Tabelle 1 unter A zu entnehmen. Im Vergleich zu Beispiel 1 ergibt sich eine deutliche Verschlechterung der Ausbeute an Wertprodukt.

In der nachfolgenden Tabelle 1 sind die Ergebnisse der gaschromatographischen Analyse (Probenahme unmittelbar nach Ende des Einpumpens) für die Beispiele 1 bis 5 und Vergleichsversuch A zu entnehmen. Die Tabelle 2 enthält die Ergebnisse der Beispiele 2 bis 5 nach jeweils 1 Stunde Nachreaktion.

**Tabelle 1**

| Beispiel | 1 | 2 | 3 | 4 | 5 | A |
|---|---|---|---|---|---|---|
| Zusammensetzung*) | | | | | | |
| n-Octylamin | - | <0,1 | <0,1 | <0,1 | - | <0,1 |
| N-Methyl-n-octylamin | | 0,2 | 0,9 | 1,1 | 2,3 | 1,1 |
| N,N-Dimethyl-n-octylamin | 93,1 | 93,3 | 96,2 | 98,1 | 96,7 | 78,26 |
| N,N-Dioctyl-methylamin | 2,5 | 4,6 | 1,0 | - | 0,3 | 15,9 |

| | | | | | | |
|---|---|---|---|---|---|---|
| *) alle Angaben in Gew.-%; H₂O und Methanol herausgerechnet. | | | | | | |

**Tabelle 2**

| Beispiel | 2 | 3 | 4 | 5 |
|---|---|---|---|---|
| Zusammensetzung*) | | | | |
| n-Octylamin | - | - | - | - |
| N-Methyl-n-octylamin | 0,2 | 0,5 | 0,6 | 1,1 |
| N,N-Dimethyl-n-octylamin | 93,7 | 96,7 | 98,4 | 97,8 |
| N,N-Dioctyl-methylamin | 4,7 | 0,7 | - | 0,1 |

| | | | | |
|---|---|---|---|---|
| *) alle Angaben in Gew.-%; H₂O und Methanol herausgerechnet. | | | | |

### Beispiel 6:

Es wird analog Beispiel 1 gearbeitet.

Nach Erreichen der Sollbedingungen werden 185 g (1 mol) n-Dodecylamin und 138g der in Beispiel 1 verwendeten Formaldehydlösung - entsprechend 2,5 mol Formaldehyd über die beiden Tauchstutzen getrennt, aber gleichzeitig über einen Zeitraum von 2 Stunden eingepumpt. Das Reaktionsgemisch enthält 0,6 Gew.-% N-Methyl-n-dodecylamin, 93,6 Gew.-% N,N-Dimethyl-n-dodecylamin und 0,6 Gew.-% N,N-Didodecylmethylamin (gaschromatographische Analyse; H₂O und Methanol herausgerechnet).

### Vergleichsversuch B

Es wird analog Vergleichsversuch A gearbeitet, jedoch statt n-Octylamin werden 185 g (1 mol) n-Dodecylamin und 3 g des in Beispiel 1 verwendeten Nickelkatalysators vorgelegt. Die Zugabe von 138 g Formaldehydlösung erfolgt wie in Vergleichsversuch A beschrieben.

Das Reaktionsgemisch besteht aus 2 Phasen, deren untere aus 89,7 Gew.-% Wasser und 10,3 Gew.-% Methanol besteht. Die oberen, organische Phase enthält 0,9 Gew.-% N-Methyl-n-dodecylamin, 80,3 Gew.-% N,N-Dimethyl-n-dodecylamin und 16,7 Gew.-% N,N-Didodecylmethylamin (gaschromatographische Analyse; H₂O und Methanol herausgerechnet)

Im Vergleich zu Beispiel 6 ergibt sich eine deutliche Verschlechterung der Ausbeute an Wertprodukt.

## Patentansprüche

1. Verfahren zur Herstellung von tertiären N,N-Dimethylaminen durch Umsetzung von primären Aminen mit Formaldehyd und Wasserstoff als Ausgangsstoffe unter Druck und erhöhter Temperatur an einem Nickel enthaltenden Hydrierkatalysator in flüssiger Phase, dadurch gekennzeichnet, daß der Hydrierkatalysator in einem Lösungsmittel suspendiert wird, die Nickel-Konzentration 0,1 bis 10 Gew.-% bezogen auf einzusetzendes primäres Amin, beträgt, die Ausgangsstoffe bei 80 bis 150 °C und 1 bis 15 MPa voneinander getrennt, aber gleichzeitig unter Vermischung in die Katalysatorsuspension eingeleitet und in einem Reaktionsschritt zu tertiären N,N-Dimethylaminen umgesetzt werden.

2. Verfahren nach Anspruch 1, dadurch gekennzeichnet, daß die Ausgangsstoffe bei 90 bis 130, insbesondere bei 95 bis 125, bevorzugt bei 100 bis 120 °C in die Katalysatorsuspension eingeleitet und umgesetzt werden.

3. Verfahren nach einem oder mehreren der Ansprüche 1 bis 2, dadurch gekennzeichnet, daß die Ausgangsstoffe bei 1,5 bis 12 MPa, insbesondere 3 bis 10 MPa, in die Katalysatorsuspension eingeleitet und umgesetzt werden.

4. Verfahren nach einem oder mehreren der Ansprüche 1 bis 3, dadurch gekennzeichnet, daß die Konzentration an Nickel 0,2 bis 5, insbesondere 0,3 bis 2,0, bevorzugt 0,6 bis 1,3 Gew.-% Nickel, bezogen auf einzusetzendes primäres Amin, beträgt.

5. Verfahren nach einem oder mehreren der Ansprüche 1 bis 4, dadurch gekennzeichnet, daß der Katalysator 20 bis 80, insbesondere 40 bis 70, bevorzugt 50 bis 65 Gew.-% Nickel enthält.

6. Verfahren nach einem oder mehreren der Ansprüche 1 bis 5, dadurch gekennzeichnet, daß der Nickel enthaltende Katalysator Al₂O₃ SiO₂, Kieselerde, Kieselgel, Aktivkohle und/oder Bimsstein als Träger aufweist.

7. Verfahren nach einem oder mehreren der Ansprüche 1 bis 5, dadurch gekennzeichnet, daß der Nickel enthaltende Katalysator Al₂O₃, SiO₂, Kieselerde und/oder Kieselgel als Träger aufweist.

8. Verfahren nach einem oder mehreren der Ansprüche 1 bis 5, dadurch gekennzeichnet, daß der Nickel enthaltende Katalysator SiO₂, Kieselerde und/oder Kieselgel als Träger aufweist.

9. Verfahren nach einem oder mehreren der Ansprüche 1 bis 8, dadurch gekennzeichnet, daß als primäres Amin geradkettige und/oder verzweigte aliphatische Amine mit 4 bis 24, insbesondere 6 bis 20, bevorzugt 8 bis 16 Kohlenstoffatomen eingesetzt werden.

10. Verfahren nach einem oder mehreren der Ansprüche 1 bis 9, dadurch gekennzeichnet, daß als Lösungsmittel das Reaktionsprodukt, aliphatische, cycloaliphatische, aromatische Kohlenwasserstoffe, Ether und/oder Alkohole eingesetzt werden.

11. Verfahren nach einem oder mehreren der Ansprüche 1 bis 9, dadurch gekennzeichnet, daß als Lösungsmittel cyclische Ether und/oder aliphatische Alkohole eingesetzt werden.

12. Verfahren nach einem oder mehreren der Ansprüche 1 bis 9, dadurch gekennzeichnet, daß als Lösungsmittel Tetrahydrofuran, Dioxan und/oder aliphatische Alkohole mit 1 bis 6 Kohlenstoffatsen, insbesondere Methanol, Ethanol, Propanol, i-Propanol, n-Butanol und/oder i-Butanol eingesetzt werden.

13. Verfahren nach einem oder mehreren der Ansprüche 1 bis 9, dadurch gekennzeichnet, daß als Lösungsmittel Methanol eingesetzt wird.

14. Verfahren nach einem oder mehreren der Ansprüche 1 bis 13, dadurch gekennzeichnet, daß der Anteil des Lösungsmittels mindestens 5 Vol.-% bezogen auf das Volumen aller flüssigen Ausgangsstoffe, beträgt.

15. Verfahren nach einem oder mehreren der Ansprüche 1 bis 14, dadurch gekennzeichnet, daß der Anteil des Lösungsmittels 5 bis 40, insbesondere 10 bis 30, bevorzugt 15 bis 25 Vol.-%, bezogen auf das Volumen aller flüssigen Ausgangsstoffe, beträgt.

16. Verfahren nach einem oder mehreren der Ansprüche 1 bis 15, dadurch gekennzeichnet, daß Formaldehyd als Lösung, die außer Formaldehyd 5 bis 15 Gew.-% Wasser und 25 bis 55 Gew.-% eines aliphatischen Alkohols, insbesondere Methanol enthält, eingesetzt wird.

17. Verfahren nach einem oder mehreren der Ansprüche 1 bis 15, dadurch gekennzeichnet, daß Formaldehyd als Lösung, die außer Formaldehyd 7 bis 12 Gew.-% Wasser und 30 bis 40 Gew.-% eines aliphatischen Alkohols insbesondere Methanol enthält, eingesetzt werden.

## Claims

1. Process for the preparation of tertiary N,N-dimethylamines by the reaction of primary amines with formaldehyde and hydrogen as starting materials under pressure and at elevated temperature on a nickel-containing hydrogenation catalyst in liquid phase, characterised in that the hydrogenation catalyst is suspended in a solvent, the nickel concentration is 0.1 to 10 % by weight, related to the primary amine to be used, the starting materials are separated from each other at 80 to 150°C and 1 to 15 MPa but are fed into the catalyst suspension at the same time with mixing and reacted in one reaction step to form tertiary N,N-dimethylamines.

2. Process according to claim 1, characterised in that the starting materials are introduced into the catalyst suspension and reacted at 90 to 130, particularly 95 to 125, preferably 100 to 120°C.

3. Process according to one or both of the claims 1 to 2, characterised in that the starting materials are introduced into the catalyst suspension and reacted at 1.5 to 12 MPa, in particular 3 to 10 MPa.

4. Process according to one or more of the claims 1 to 3, characterised in that the concentration of nickel is 0.2 to 5, in particular 0.3 to 2.0, preferably 0.6 to 1.3 % by weight, related to the primary amine to be used.

5. Process according to one or more of the claims 1 to 4, characterised in that the catalyst contains 20 to 80, in particular 40 to 70, preferably 50 to 65 % by weight of nickel.

6. Process according to one or more of the claims 1 to 5, characterised in that the nickel-containing catalyst exhibits Al₂O₃, SiO₂, siliceous earth, silica gel, activated carbon and/or pumice stone as carriers.

7. Process according to one or more of the claims 1 to 5, characterised in that the nickel-containing catalyst exhibits Al₂O₃, SiO₂, siliceous earth, and/or silica gel as carriers.

8. Process according to one or more of the claims 1 to 5, characterised in that the nickel-containing catalyst exhibits SiO₂, siliceous earth and/or silica gel as carriers.

9. Process according to one or more of the claims 1 to 8, characterised in that straight-chain and/or branched aliphatic amines having 4 to 24, in particular 6 to 20, preferably 8 to 16 carbon atoms are used as the primary amine.

10. Process according to one or more of the claims 1 to 9, characterised in that the reaction product, aliphatic, cycloaliphatic, aromatic hydrocarbons, ether and/or alcohols are used as solvents.

11. Process according to one or more of the claims 1 to 9, characterised in that cyclic ether and/or aliphatic alcohols are used as solvents.

12. Process according to one or more of the claims 1 to 9, characterised in that tetrahydrofuran, dioxane and/or aliphatic alcohols having 1 to 6 carbon atoms, especially methanol, ethanol, propanol, i-propanol, n-butanol and/or i-butanol are used as solvents.

13. Process according to one or more of the claims 1 to 9, characterised in that methanol is used as the solvent.

14. Process according to one or more of the claims 1 to 13, characterised in that the proportion of the solvent is at least 5 % by volume, related to the volume of all the liquid starting materials.

15. Process according to one or more of the claims 1 to 14, characterised in that the proportion of the solvent is 5 to 40, in particular 10 to 30, preferably 15 to 25 % by volume, related to the volume of all the liquid starting materials.

16. Process according to one or more of the claims 1 to 15, characterised in that formaldehyde is used as a solution, which apart from formaldehyde contains 5 to 15 % by weight of water and 25 to 55 % by weight of an aliphatic alcohol, in particular methanol.

17. Process according to one or more of the claims 1 to 15, characterised in that formaldehyde is used as a solution, which apart from formaldehyde contains 7 to 12 % by weight of water and 30 to 40 % by weight of an aliphatic alcohol, in particular methanol.

## Revendications

1. Procédé de préparation de N,N-diméthylamines tertiaires par réaction d'amines primaires avec le formaldéhyde et l'hydrogène servant de produits de départ, sous pression et à température élevée, sur un catalyseur d'hydrogénation contenant du nickel, en phase liquide, caractérisé en ce que l'on met le catalyseur d' hydrogénation en suspension dans un solvant, la concentration en nickel est de 0,1 à 10 % du poids de l'amine primaire mise en oeuvre, les composants de départ sont injectés dans la suspension de catalyseur séparément mais en même temps et mélangés à des températures de 80 à 150°C et des pressions de 1 à 15 MPa, et convertis en un stade de réaction en N,N-diméthylamines tertiaires.

2. Procédé selon la revendication 1, caractérisé en ce que les composants de départ sont injectés dans la suspension de catalyseur où ils réagissent à des températures de 90 à 130, de préférence de 95 à 125 et plus spécialement de 100 à 120°C.

3. Procédé selon une ou plusieurs des revendications 1 à 2, caractérisé en ce que les composants de départ sont injectés dans la suspension de catalyseur où ils réagissent à des pressions de 1,5 à 12 MPa, plus spécialement de 3 à 10 MPa.

4. Procédé selon une ou plusieurs des revendications 1 à 3, caractérisé en ce que la concentration en nickel est de 0,2 à 5, de préférence de 0,3 à 2,0 et plus spécialement de 0,6 à 1,3 % en poids de nickel par rapport à l'amine primaire mise en oeuvre.

5. Procédé selon une ou plusieurs des revendications 1 à 4, caractérisé en ce que le catalyseur contient de 20 à 80, plus spécialement de 40 à 70, de préférence de 50 à 65 % en poids de nickel.

6. Procédé selon une ou plusieurs des revendications 1 à 5, caractérisé en ce que le catalyseur contenant du nickel comprend un support consistant en alumine, silice, gel de silice, charbon actif et/ou ponce.

7. Procédé selon une ou plusieurs des revendications 1 à 5, caractérisé en ce que le catalyseur contenant du nickel comprend un support consistant en alumine, silice et/ou gel de silice.

8. Procédé selon une ou plusieurs des revendications 1 à 5, caractérisé en ce que le catalyseur contenant du nickel comprend un support consistant en silice et/ou gel de silice.

9. Procédé selon une ou plusieurs des revendications 1 à 8, caractérisé en ce que les amines primaires mises en oeuvre sont des amines aliphatiques à chaîne droite et/ou ramifiée en C₄-C₂₄, plus spécialement en C₆-C₂₀, de préférence en C₈-C₁₆.

10. Procédé selon une ou plusieurs des revendications 1 à 9, caractérisé en ce que l'on utilise en tant que solvants le produit de réaction lui-même, des hydrocarbures, éthers et/ou alcools aliphatiques, cycloaliphatiques ou aromatiques.

11. Procédé selon une ou plusieurs des revendications 1 à 9, caractérisé en ce que l'on utilise en tant que solvants des éthers cycliques et/ou des alcools aliphatiques.

12. Procédé selon une ou plusieurs des revendications 1 à 9, caractérisé en ce que l'on utilise en tant que solvants le tétrahydrofuranne, le dioxanne et/ou des alcools aliphatiques en C₁-C₆, en particulier le méthanol, l'éthanol, le propanol, l'isopropanol, le n-butanol et/ou l'isobutanol.

13. Procédé selon une ou plusieurs des revendications 1 à 9, caractérisé en ce que l'on utilise en tant que solvant le méthanol.

14. Procédé selon une ou plusieurs des revendications 1 à 13, caractérisé en ce que la proportion de solvant est d'au moins 5 % en volume par rapport au volume de tous les composants de départ liquides.

15. Procédé selon une ou plusieurs des revendications 1 à 14, caractérisé en ce que la proportion de solvant est de 5 à 40, plus spécialement de 10 à 30 et de préférence de 15 à 25 % en volume par rapport au volume de tous les composants de départ liquides.

16. Procédé selon une ou plusieurs des revendications 1 à 15, caractérisé en ce que le formaldéhyde est mis en oeuvre à l'état de solution contenant, outre le formaldéhyde, 5 à 15 % d'eau, et 25 à 55 % en poids d'un alcool aliphatique, en particulier le méthanol.

17. Procédé selon une ou plusieurs des revendications 1 à 15, caractérisé en ce que le formaldéhyde est mis en oeuvre à l'état de solution contenant, outre le formaldéhyde, de 7 à 12 % en poids d'eau et de 30 à 40 % en poids d'un alcool aliphatique, en particulier le méthanol.
